# EUROPEAN PATENT APPLICATION

(11) **EP 4 456 082 A1**
(43) Date of publication of application: **30.10.2024**
(21) Application number: 23315102.6
(22) Date of filing: 25.04.2023
(51) Int. Cl.: G16H 40/60, G16H 50/20

(54) **A SYSTEM FOR DETERMINING A DYNAMIC EXAMINATION PROCESS**

(71) Applicant: SUPERSONIC IMAGINE, 13290 Aix-en-Provence (FR)
(72) Inventor: SPORTOUCHE, Hélène, 13009 Marseille (FR); HENRY, Jean-Pierre, 13001 Marseille (FR)
(74) Representative: Paustian & Partner Patentanwälte mbB

(57) **Abstract**

The invention relates to a system (10) for determining a dynamic examination process, comprising a processing unit (30) configured to:
determine a dynamic examination process (100) based on a pathology hypothesis using a predefined algorithm,
the dynamic examination process (100) being associated with obtaining medical data,
wherein the dynamic examination process (100) is adaptable during its execution based on the obtained medical data.

## Description

### BACKGROUND

In the field of medicine, the use of predefined protocols can improve the quality of clinical outcomes, such as for example the efficiency of medical examination data of a subject. A system for obtaining medical data, for example an ultrasound imaging system, may be provided with a set of "factory" protocols for predefined applications. A "factory" protocol may be programmed by the manufacturer of the system and be pre-installed on the system or predefined by a particular user for a group of users. Such types of predefined protocols may for example concern examination of a human organ, such as a liver or a breast.

One advantage of providing such protocols is that even novice users can use the system in an efficient way, following the predefined associated protocol. For example, when examining the liver of a subject, the user may select the "liver" protocol and then needs to perform the examination steps specified by said protocol (for example, selecting a probe, using predefined parameters of the medical device, holding an ultrasound probe at a particular location of a subject, pressing a "freeze" button or other buttons of the medical device, etc.)..

However, a disadvantage of this method is that the factory protocols have been customized by the manufacturer or the user for a particular system, and/or for a particular application or organ scan but not for a particular subject to be examined (i.e., in the current example a patient). Thus, although the predefined protocols provide for specific applications (in particular, examinations of specific organs), they cannot address the specificities of the current scanned subject.

### SUMMARY OF THE DISCLOSURE

A system for determining a dynamic examination process is provided. The system comprises a processing unit configured to determine a dynamic examination process based on a pathology hypothesis using a predefined algorithm. Furthermore, the dynamic examination process (for example when executed) is associated with obtaining clinical data. The dynamic examination process is advantageously adaptable during its execution in particular based on the obtained medical data.

By providing such a system, a dynamic examination process can be achieved. In this context, the examination process may be dynamic by being adaptable (i.e. dynamically adaptable) during its execution. Accordingly, the examination process is not necessarily fully predetermined but may still evolve during its execution. The adaptations of the examination process, i.e. its dynamic behavior, may be thereby controlled or steered by the obtained medical data.

For example, the system may be configured to automatically adapt the dynamic examination process during its execution.

Before starting the execution of the dynamic examination process, an initial form of the dynamic examination process may be defined and/or determined. Said initial form may be determined, or built or selected, based on a medical or pathology hypothesis. Accordingly, the dynamic examination process can be adaptive to a pathology hypothesis of a subject (for example a patient). Hence, the basic or initial form of the dynamic examination process may already be controlled by or adapted to characteristics of the particular subject. Moreover, since the dynamic examination process can be adapted during its execution based on newly obtained medical data of the subject, the dynamic examination process may be further customized or adapted to the particular characteristics of the subject (for example to pathology characteristics discovered based on obtained medical data). Subsequent stages of the examination process may for example be adapted such that particular medical data is obtained in these added stages, which allow a verification, confirmation and/or refinement of the discovered (or supposed) pathology characteristics or the initial pathology assumption.

Accordingly, the examination process can be advantageously adapted and/or optimized such that the obtained medical data becomes more accurate, more comprehensive, more reliable and/or more meaningful for a particular subject. For example, the overall obtained medical data may allow to more reliably and more precisely assess, allowing the user or medical team to better understand, identify or interpret, a pathology of the subject and/or to allow verifying the initial pathology assumption or to finetune the initial assumptions or to select a pathology in a group of pathologies. This advantageously also may lead to more efficient exams, and thus conduct to save of time and minimize associated costs.

The dynamic examination process may generally define how to perform an exam, including but not limited to a set of rules and/or a set of procedures for medical examinations. The dynamic examination process may define the format and structure of data, such as subject information, diagnostic results, images, and other types of medical records. The dynamic examination process may outline the methods and/or rules for acquiring, transmitting, and/or receiving information of a subject (i.e. subject data, for example medical data). The dynamic examination process for subject examinations may improve the efficiency and accuracy of medical data management.

The dynamic examination process for medical examinations may thus improve subject outcomes, in particular in case the user of a system performing the dynamic examination process is not skilled enough to manually adapt an examination process to individual subject conditions. The dynamic examination process may hence be particularly useful for complex medical conditions or cases where multiple factors need to be considered and can affect each other.

Accordingly, the system of the present disclosure may lead to a reduction of the user dependency (novice vs expert) for data acquisition and interpretation due to the possible automation of determining the individual examination process. The system may further allow:
an increased exam reproducibility for follow-up exams over time, an increased exam standardization and documentation;
the capability to provide personalized and optimal examination with well-targeted measurements and relevant assessment of pathology biomarkers, leading to an improved accuracy of the exam outcomes;
a cost reduction (for example man power of medical experts, more generally reduction of healthcare costs) due to an improved subject management (the dynamic examination process may for example avoid irrelevant additional exams, useless interventional procedures, unnecessary follow-up exams, generally due to partial and/or improper acquisition of the data leading to potential misinterpretation of the data); and
a cost reduction due to an increased productivity, for example due to an accelerated examination process.

Examinations may be or may comprise any medical examination. Medical examinations may be part of a diagnostic process, and there may be several different types of examinations that healthcare professionals may use to assess a subject's health. A medical examination may be a general assessment of a subject's overall health that may comprise looking, listening, and touching different parts of the body. Medical examinations may comprise blood tests, urine tests, X-ray scans, magnetic resonance imaging (MRI) scans, computer tomography (CT) scans, and/or ultrasound scans. Blood tests and urine tests may comprise laboratory analyses that may provide valuable information about a subject's health status, such as the presence of infections or abnormalities in blood cell counts. Medical imaging techniques, such as X-rays, CT scans, MRI, and ultrasounds, may be used to produce detailed images of relevant part(s) of the subject, allowing healthcare professionals to identify potential health problems. These examinations may help healthcare professionals to make accurate diagnoses and/or prognoses, and to develop appropriate treatment plans to help patients to manage and recover from their medical conditions The examinations may further provide patient risk stratification.

A dynamic examination process may be provided for examinations of a subject. The term "subject" may refer to a patient who may be the focus of clinical attention, investigation, or treatment. The term "subject" may include sick or healthy humans who are receiving medical care, as well as animals who are being treated by veterinarians. A supposed healthy subject may also be examined for prophylactic or preventive purposes, i.e. to avoid potential development of any illness or pathology. A subject may also be examined during or after a medical treatment. In research studies, the term "subject" may also be used to refer to individuals or animals who are participating in the study, whether as patients or healthy volunteers. The use of the term "subject" may reflect the fact that these individuals may be the primary focus of attention and observation in the medical or research context.

Determining the dynamic examination process may be a complex process that may comprise selecting, generating, building, and/or adapting various parts of the dynamic examination process. Selecting parts of the dynamic examination process may comprise reviewing existing medical guidelines and best practices to identify appropriate diagnostic tools, treatment plans, and other clinical recommendations.

The dynamic examination process may be determined using a computer-implemented algorithm, for example an artificial intelligence (AI) based model or algorithm. In one example, the algorithm used to adapt the dynamic examination process may be the predefined algorithm to determine the dynamic examination process or may be linked to said predefined algorithm. For example, the same AI model may be used to initially determine the dynamic examination process and then to adapt it dynamically. In a further example, the algorithm used to adapt the dynamic examination process may be or may comprise a machine learning algorithm, for example a Reinforcement learning (RL) algorithm using intelligent agents ought to take actions to explore an unknown environment (i.e. the particular subject). The algorithm used to adapt the dynamic examination process may hence comprise or may involve a Markov decision process (MDP).

Generating and/or determining parts of the dynamic examination process may comprise using artificial intelligence based algorithms (for example machine learning algorithms) to analyze large sets of medical data to identify relevant patterns and trends. Building parts of the dynamic examination process may comprise creating a set of rules and decision trees that guide the user (for example a physician) based on specific subject characteristics and medical history. For example the user may be guided to follow specific steps (for example the type of acquisition and/or type of measurement) and/or to gather the relevant information leading to an accurate diagnosis and/or prognosis, and optionally to a clinical decision making, accordingly. Adapting parts of the dynamic examination process may comprise adjusting the dynamic examination process (for example in real-time and/or during the examination process) based on new research findings, subject outcomes, or changes in healthcare guidelines and best practices. Overall, determining the dynamic examination process may comprise a combination of these different approaches and may be iterative in nature as new data and insights become available (for example in real-time and/or during the examination process).

The pathology hypothesis may be a pathology hypothesis of the subject. The pathology hypothesis may be a supposed and proposed explanation for the cause and/or nature of a particular disease or disorder. The pathology hypothesis may be established by a doctor, medical specialist, physician and/or statistics, publications, and/or based on physician's empirical knowledge. For example, a particular pathology hypothesis (for instance liver fibrosis) may be subject to a particular screening program (for instance a program which evaluates liver fibrosis in a specific population). Physicians may use pathology hypotheses to guide their investigations into the underlying mechanisms of a disease, which optionally may help them to identify the appropriate treatment and management strategies. The pathology hypothesis may be provided to the predefined algorithm before generating the dynamic examination process. The pathology hypothesis may be based on symptoms of the disease(s) in question. The pathology hypothesis may also be based on knowledge of the underlying biology and physiology of the affected organs and tissues. In other words, the pathology hypothesis may be external data provided to the system. In another example, the pathology hypothesis may be determined (or predicted) by the system based on data of the subject, as described below. A determined pathology hypotheses may be revised or refined over time and/or in real-time during the execution of the examination process as new data and insights become available (i.e. new medical data is obtained).

In one aspect, the pathology hypothesis may be determined (or predicted) automatically or semi-automatically based on available data of the subject, for example using a computational tool feature. For example, determining the pathology hypothesis may be based on medical examination data of a previous examination of the subject (i.e. one example of accessible data). Furthermore, determining the pathology hypothesis may be further based on data associated with symptoms of the person. Determining the pathology hypothesis may also be based on a risk factor associated with the subject's health. Furthermore, determining the pathology hypothesis may be further based on a subject's health condition. Determining the pathology hypothesis may also be based on a subject's lifestyle.

The predefined algorithm may be a set of instructions that may be established in advance (i.e. before the execution of the dynamic examination process) and optionally used to solve a specific problem or accomplish a particular task. The predefined algorithm may be used to create the dynamic examination process. The algorithm may be designed to analyze and interpret data, such as a subject' characteristics such as age, sex, height and/or weight, subject's medical history, diagnostic results, and any other relevant clinical information.

The algorithm may then use this data to generate a dynamic examination process that may provide guidance on the exam to be performed (i.e. which operations to be followed by a user, in case the process is not executed fully automatically, for example by a robotic system), on the data to acquire (for example which mode to use), and/or on the tools to use (for example which characteristics and/or biomarkers to measure). Examples of pathology hypotheses, based in which a dynamic examination process is determined using the predefined algorithm, comprise but is not limited to liver diseases, musculoskeletal (MSK) diseases, sepsis, cancer (for instance breast cancer), and heart disease.

The term "predefined" may refer to something that may have already been established, specified, or set in advance. A predefined algorithm may have already been built, established, specified, or set before the execution of the dynamic examination process. A predefined algorithm may refer to a specific set of rules, calculations, or instructions that may have been predetermined for use in analyzing medical data and making diagnoses. A predefined algorithm may be a trained model. A model may be developed by training it on a set of data, so that it may learn patterns and relationships in the data. The resulting model may then be used to make predictions or classifications on new data.

Obtaining medical data may comprise collecting and storing information about subjects, optionally including their medical history, diagnostic results, treatment plans, and other relevant clinical data. Medical data may be obtained from a variety of sources, such as medical devices, electronic health records, medical imaging studies, laboratory test results, and/or subject-reported data. Medical data may be advantageous for healthcare providers to make informed decisions about subject care, optionally including diagnosis and/or prognosis, treatment, and management of medical conditions. Medical data may also be used for research purposes, helping to advance our understanding of disease and improve patient outcomes. Obtaining medical data and storing them may be done in a secure and confidential manner, in compliance with relevant laws and regulations. Data privacy and security may be critical concerns when obtaining medical data, and healthcare providers and organizations may implement appropriate safeguards to protect subject confidentiality and prevent data breaches. Obtaining medical data may be or may comprise the acquisition of medical data.

The dynamic examination process may be associated with obtaining medical data, wherein "associated" may mean that the dynamic examination process may be linked to or connected with the process of obtaining medical data, in particular during execution of the process. In other words, executing the process may imply obtaining medical data, but also other tasks, as for example processing and/or analyzing the medical data, generating a report, etc. (as described in detail below). The dynamic examination process may be used to guide the collection and processing of the medical data. The dynamic examination process may also (at least semi-automatically, for example with a limited assistance by a human user, who for instance holds a probe) control the process of obtaining medical data. The dynamic examination process and the collection of medical data may be interdependent, as the data may be collected according to the specifications and requirements optionally set out in the dynamic examination process.

The dynamic examination process may be adaptable during its execution based on the medical data obtained during the subject's examination. As medical data may be obtained, dynamic examination process may be designed to analyze and/or optionally interpret the data in real-time, optionally allowing for dynamic adjustments to the subject's diagnosis, treatment, and/or care plan. For example, if a subject's results reveal complications or so far unknown symptoms, the dynamic examination process may automatically adapt to provide new recommendations and guidance based on the latest medical evidence and best practices. By leveraging the power of artificial intelligence, the dynamic examination process may provide personalized and responsive healthcare that may optionally be tailored to the individual subject's needs and pathologies. In other words, the dynamic examination process may be adapted, customized, personalized or subject-specific. The adaptable dynamic examination process may increase the accuracy of the diagnostics and/or prognostics. The adaptable dynamic examination process may further improve the quality and efficiency of healthcare by minimizing errors, optionally reducing unnecessary procedures and treatments, and enhancing subject outcomes. As medical data may continue to be collected and analyzed, dynamic examination process may possibly become even more effective in providing precise and personalized care to subjects. It may be possible that the dynamic examination process may be adaptable during execution based on other criteria, such as subject-specific factors or external factors. Subject-specific factors may be, for example, the subject's age, medical history, symptoms, and response to treatment. Externals factors may be, for example, changes in healthcare regulations, new medical research findings, or the availability of new medical treatments or therapies. Furthermore, other criteria may be, for example, personal preferences, cultural or religious considerations.

The dynamic examination process may be computer implemented. The dynamic examination process may comprise a step, a sequence of steps and/or a stage or several stages. The dynamic examination process may comprise operations. In other words, a step and/or stage may be associated with a task or a group of tasks, such as obtaining data.

For example, the dynamic examination process may be a set of instructions and guidelines that may be programmed into a computer system and used to carry out a specific task or process. The dynamic examination process may be an at least partially automated workflow. Accordingly, at least some operations of the examination process may be carried out by a system. Other operations may be guided and/or controlled by the system but may require assistance by a user (for example by holding an ultrasound probe). Anyway, the user is advantageously much less required to have particular skills due to its guidance and/or control.

In one aspect, the dynamic examination process may define at least one entity capable of executing or controlling (or assisting) a task.

The entity may refer to a specific unit, component and/or element within the dynamic examination process. The entity may represent a particular procedure, concept, storage and/or data point. Examples of data points within a dynamic examination process may comprise subject demographic information, medical history, vital signs, laboratory test results, diagnostic imaging data, treatment plans, and/or medication orders. A further example of an entity may include a particular procedure, for example a procedure for adjusting and/or fine-tuning of diagnostic imaging data, a procedure for exploitation of at least one of: diagnostic data, a measurement of diagnostic parameters, an assessment of diagnostics descriptors.

An entity may comprise a set of rules and/or a set of procedures to execute a task. An entity may hence be configured to execute a task. An entity may however also be configured to control or assist a task or execution of a task. For example, the task may at least partially be executed by a user (or an external device or system) under the control or assistance of the entity. The entity may also comprise information that may be relevant to conduct the dynamic examination process. Entities within a dynamic examination process may be organized into a standardized structure, such as a database or electronic health record system, which may allow for easy retrieval and analysis of the information they contain. This structure may also enable the use of machine learning algorithms and other computational tools to analyze large sets of medical data and identify relevant patterns and trends that may guide clinical decision-making. Entities within a dynamic examination process may be advantageous, as they may enable for example healthcare providers to collect, store, and/or analyze subject data in a standardized way, which may lead to more accurate diagnoses, more effective treatment plans, improved subject outcomes, more efficient and reproducible exams, and a higher productivity.

An entity may be, for example, an intelligent component, for example an analyzing and/or processing component. The component may be or may comprise a software module. The component may thus be software based. The component may for instance comprise or be an artificial intelligence (AI) based algorithm. The component may be hence a particular intelligent type of a computational and/or processing tool, as described above, and thus may also be referred to as "intelligent component". Accordingly, the intelligent component may differ from other conventional tools by incorporating an algorithm (for example an AI-based algorithm) which equips the component with a certain level of intelligence or comprehensibility of a particular task to be handled by the component.

A component may for example be configured to analyze data and may thus be referred to as for example an analyzing component. For example, the component may be configured to derive information of the subject based on obtained medical data. The information may for example insights into the subject condition, for instance for evaluating a pathology of the subject. The component may for example process various medical parameters related to an examination, such as biomarkers, measurement parameters, or other relevant data. The component may analyze this data to produce an output, which may be in the form of another biomarker or a pathology scoring, grading, and/or assessment, optionally related to the input biomarker.

A component may also be configured to control the examination process and may thus be referred to as for example a processing component. For example, the component may automatize the measurement of a given biomarker. For instance, the measurement may be used as input of another component (for example focusing on data analysis)

The selection of components in the dynamic examination process and/or a property of a used component may be adaptable during the execution of the dynamic examination process based on the medical data obtained, for example in real-time. The means a component may for example adjust its processing techniques or criteria depending on the specific needs related to the current subject.

In one aspect, the at least one entity may be adaptable during execution of the dynamic examination process based on the obtained medical data.

For example, if a medical device may be used to measure a subject's blood pressure during a medical examination, an entity of the dynamic examination process may analyze the obtained medical data and determine that the subject's blood pressure is outside of the expected range for their age and health status. In response, the entity of the dynamic examination process may adapt by adding a step or stage to the process to address the high blood pressure such as doppler measurement or organ elasticity.

An adaptable entity in a dynamic examination process may refer to an entity that may be to adapt its behavior or features in response to changing circumstances or inputs. This may involve adding, removing, adjusting, or replaying certain actions or steps or stages of the entities. This may also involve adapting some parameters of the device used to perform some steps or stages of the exam. Entities may also be added or removed. Adaptation may be based on the results of previous examinations, based on data acquired in 'real-time' or pseudo-real time, or other relevant data. Training data may comprise knowledge about adaptability made by an expert in previous examinations. Training data may be used to inform the behavior of such adaptable entities in the dynamic examination process. By analyzing past cases and outcomes, the system may learn how to adjust its approach to achieve better results in the future. This may involve modifying the selection of types of data to acquire, changing the selection of probes or modes, adjusting measurement tools, or other related factors.

In one aspect, the task and/or the entity may be associated with at least one of: biomarkers (for example with obtaining a value of a particular biomarker of the subject), computational tools, measurement tasks, and/or measurement values, acquisition modes, probe types, machine parameters, machine settings, scan parameters, acquisition process for obtaining medical data, processing tools, reporting tools, and selecting medical data.

The biomarker values computed for the subject may be determined based on the medical data. The computational tools may be used, for example, for processing the acquired data or assisting a user for obtaining the medical data and/or scanning the subject. Measurements may be based on or using the medical data. Measurements may be automatically and/or manually conducted. Furthermore, measurements may be done on images displayed on a screen. Acquisition modes may be a set of parameters and algorithms, optionally allowing different types of data (for example with different physical meanings) to be obtained, computed and/or displayed.

An acquisition mode may be, for example, B-mode or ShearWave^{™} Elastography (SWE). Probe types may be used for obtaining the medical data. Machine parameters and/or settings (for instance controlled by the user) may be used for controlling a medical device for obtaining the medical data. An acquisition process for obtaining medical data may be used, for example, according to a particular acquisition mode and/or with a particular probe. Processing tools may be used for processing obtained medical data. A selection of medical data may be or may comprise, for example, the selection of an image. Furthermore, the task and/or the entity may be associated with an aid to diagnosis, aid to prognosis, decision support and/or a second opinion.

Machine parameters and/or settings (for instance controlled by the user) for an ultrasound machine may comprise settings such as mode, gain, depth, frequency, focus, dynamic range, persistence, compression, harmonic imaging, spatial compounding, speckle reduction, edge enhancement, color Doppler settings, pulsed wave Doppler settings, and power Doppler settings, among others.

Furthermore, the task and/or the entity may be associated with the selection of a particular probe, clinical guidelines, best practice recommendations, and/or medical research findings.

In one aspect, the dynamic examination process may define at least one predefined acquisition mode of the medical device the acquisition mode being associated with a group of entities.

In other words, at least one entity or a group of entities may be associated with a predefined acquisition mode of the medical device. The predefined acquisition mode may be or may comprise a predefined examination mode.

In one aspect, adapting the dynamic examination process may comprise, based on the medical data obtained by a first entity or a first group of entities, reexecuting the first entity or first group of entities using at least one adapted machine parameter. Furthermore, adapting the dynamic examination process may comprise, based on medical data obtained by a first entity or first group of entities, selecting and/or adapting a second entity or a second group of entities. Furthermore, the first and second group of entities may be associated with different acquisition modes.

In one aspect, adapting the second entity may comprise at least removing the second entity from the dynamic examination process. Furthermore, adapting the second entity may comprise at least adding the second entity to the dynamic examination process. Adapting the second entity may also comprise at least adapting at least one parameter of the second entity, for example a machine parameter or another characteristic of the second entity. For instance, such another characteristic may also characterize the context of the second entity, for example a used mode or algorithm associated with the second entity.

In one aspect, adapting the second group of entities may comprise adding a second group of entities to the dynamic examination process. Furthermore, adapting the second group of entities may comprise at least selecting a second group of entities of the dynamic examination process to be executed. Adapting the second group of entities may also comprise at least removing the second group from the dynamic examination process. Furthermore, adapting the second group of entities may comprise at least deselecting the second group from being to, be executed. Furthermore, adapting the second group of entities may comprise at least fine-tuning the second group of entities.

Fine-tuning may refer to the process of making small adjustments or modifications to an existing model or system in order to improve its relevance and/or performances. In the context of the dynamic examination process, fine-tuning may refer to making changes to the second group of entities in order to better suit a specific examination or pathology hypothesis. These changes may include adding or removing entities, adjusting the parameters of existing entities, or modifying the communication channels between entities.

In one aspect, at least one entity may be executable using a medical device.

Examples of medical devices include magnetic resonance imaging (MRI) scanners, computed tomography (CT) scanners, X-ray machines, ultrasound machines, dialysis machines, blood pressure monitors, thermometers, blood glucose meters, infusion pumps, ventilators, electrocardiogram (ECG) machines, and pulse oximeters.

In one aspect, the dynamic examination process may define a group of interconnected entities, wherein at least two entities of the group may depend on each other.

In a group of interconnected entities, the entities may depend on each other. The actions or output of one entity may, for example, influence or be influenced by another entity in the group. For example, in a dynamic examination process for medical examinations, the data obtained from one examination may influence the decision-making process for the subsequent examination. Similarly, in an AI algorithm for medical diagnosis, the output of one stage of the algorithm may be used as input for the next stage. The degree of interdependence between entities in a group may vary, and some entities may be more impactful than others in terms of their influence on the overall performance of the group.

For example, in a medical device, choosing a suitable acquisition mode may be critical for obtaining meaningful and accurate information of the examined subject, whereas a contrast setting of a displayed image may be less crucial. Understanding the interdependence between entities in a group may be important for designing and optimizing the performance of the group as a whole. Examples of interdependence between entities may be or may comprise correlation, sequential dependency, hierarchical dependency, and/or feedback loops.

A dependency between two entities may be determined based on confounding factors between the entities. Also, it some cases, the interdependency between two entities may be exploited to improve the outcomes of one of these two entities or coming of a third entity. As a first example, if the first entity is an imaging biomarker and the other entity is another imaging biomarker, and if the value of the first biomarker influences the value of the second biomarker, it may be possible to leverage this interdependency to refine the outcome of a third entity, whose objective is to assess a specific disease taking into account the two values of these biomarkers. As a second example, if the first entity corresponds to the data coming from a given imaging mode and if the second entity corresponds to the data coming from another imaging mode, it might be possible to use the data acquired with the second mode to correct the values of the data associated with the first imaging mode. As a third example, if the first entity corresponds to the biomarker coming from a given imaging mode and if the second entity corresponds to another biomarker coming from a second imaging mode (which could be the same mode like the first mode or another), it might be possible to use the value(s) of the biomarker coming from the second mode to correct the value(s) of the biomarker coming from the first imaging mode.

In one aspect, at least two entities of the group of interconnected entities may be capable of communicating with each other through a digital communication channel optionally established according to the dynamic examination process.

The digital communication channel may be a medium through which entities may exchange information with each other. There are many types of digital communication channels, such as wired and wireless channels, serial and parallel channels, and synchronous and asynchronous channels. A digital communication channel may be or may comprise the internet, wireless networks, or specialized medical data networks. The choice of communication channel may depend on factors such as the speed and reliability of the channel, the distance between the entities, and the amount of data that needs to be transmitted. A digital communication channel may also be a virtual channel. For example, in case two entities are both software programs, they may also communicate via a communication channel in the form of a program interface (such as an API). Accordingly, the communication channel may also comprise for example a data interface or an application programming interface.

For example, in a medical device that may include sensors, a processing unit, and a display, the sensors may be connected to the processing unit using a wired communication channel such as a USB cable. The processing unit may then transmit data to the display using a wireless communication channel such as Bluetooth^{®}.

In one aspect, the dynamic examination process may organize at least a part of the group of interconnected entities.

The dynamic examination process may organize at least a part of the group of interconnected entities according to an architecture selected from the group consisting of a sequential architecture, a parallel architecture, a hierarchical architecture, and a distributed architecture. The dynamic examination process may be organized as a network or as a web of interconnected entities.

A sequential architecture may involve organizing entities in a linear fashion, with one entity following another in a predetermined order. A sequential architecture may be used for processes that may have a clear and predictable sequence of stages, such as medical procedures or treatment processes. A parallel architecture may involve organizing entities in a way that may allow multiple processes to occur simultaneously. A parallel architecture may be used for complex medical procedures or tests that require the simultaneous processing of multiple data points. A hierarchical architecture may involve organizing entities in a tree-like structure, with smaller subgroups of entities grouped together under larger categories. A hierarchical architecture may be used for organizing medical data in a way that makes it easier to navigate and search for specific information. A distributed architecture may involve organizing entities in a way that may allow them to be stored and accessed across multiple locations or devices. A distributed architecture may be used for medical data that may need to be accessed by multiple healthcare providers or medical facilities.

The dynamic examination process may organize a first part of the group of interconnected entities according to a first architecture and a second part of the group of interconnected entities according to a second architecture. The choice of architecture for organizing entities within a dynamic examination process may depend on the specific needs and requirements of the clinical scenario.

In one aspect, the architecture may be adaptable during execution of the dynamic examination process based on the obtained medical data including an adaptation of interconnections and/or communication channels.

For example, if new information may be obtained during execution of the dynamic examination process, the architecture may adapt by dynamically changing the communication channels between different entities to ensure that the updated information is properly transferred, processed and/or analyzed. This dynamic adaptation of the architecture may help to ensure that the dynamic examination process remains efficient and effective in achieving its intended goals, even in the face of changing or uncertain medical data.

A risk factor of the subject's health may be for example the genetic factors, the age of the subject. A subject's health condition may comprise or may be defined by diabetes, for example. A subject's lifestyle may comprise or may be defined by junk food fan, smoking person, sedentary, for example.

In one aspect, the obtained medical data may comprise data acquired from the subject in real-time. Furthermore, the obtained medical data may comprise at least medical examination data. The obtained medical data may also comprise at least data acquired by at least one medical device, optionally including an ultrasound system.

In one aspect, the dynamic examination process may be adaptable in real-time during the digital protocol execution and/or during the ongoing medical examination.

The dynamic examination process may be adaptable in real-time or pseudo real time based on the data it receives during execution. The dynamic examination process may be designed to monitor a subject's condition and adjust the treatment plan in real-time based on changes in the subject's health. For example, if the subject's vital signs change suddenly, the dynamic examination process may modify the treatment plan to address the new conditions. Real-time adaptability may be achieved through the use of fast algorithms, including for example pretrained and customized ML algorithms or other forms of AI algorithms. Such algorithms may analyze data from sensors, medical devices, and other sources to detect patterns and optionally make predictions about the subject's health in real-time. The system may then use this information to adapt the examination process dynamically, in order to adjust which medical data is still to be obtained and optionally to modify accordingly the diagnostics / prognostics tools to use. Subsequently, the treatment plan, adjust dosages of medication, or other actions may be modified, respectively, to optimize the subject's care.

The real-time adaptation may be implemented such that a user of the system is not or less distracted by any adaption. For example, the user may be merely informed (for example via a user interface, for example a display) of the subsequent step or stage (or a limited number of subsequent steps or stages) to be executed and optionally about an estimated time to complete the complete examination process. Hence, modifications concerning other steps or stages may be made in the background without distracting the user.

In one aspect, the dynamic examination process may define at least one entity configured to estimate a biomarker of the subject based on obtained medical data of the subject. Furthermore, the dynamic examination process may define at least one entity configured to assess the pathology hypothesis based on one or several estimated biomarkers.

In one aspect, the pathology hypothesis may comprise a hypothesis of a pathologies family. Furthermore, the dynamic examination process may define at least one entity configured to assess the pathology hypothesis or a particular member of a pathologies family as a function of the obtained medical data.

In one aspect, the predefined algorithm may be or may comprise an artificial intelligence (AI) model.

In a further aspect, at least one entity may be or may comprises an artificial intelligence (AI) model.

Examples of predefined algorithms may comprise AI models, such as decision trees, random forests, logistic regression, gradient boosting, and/or support vector machines. Predefined algorithms may be used to perform classification and prediction tasks, such as image recognition. For example, a decision tree algorithm may be used to classify images of objects based on their features. Other examples of predefined algorithms may comprise neural networks, k-nearest neighbors, and/or clustering algorithms, which may be used for tasks such as anomaly detection and data segmentation. AI may refer to the development of computer systems and algorithms that may perform tasks that typically require human intelligence, such as visual perception, speech recognition, decision-making, and language translation. AI systems and algorithms may be trained using large sets of data and sophisticated algorithms that may allow them to identify patterns and trends, make predictions, and optimize outcomes.

The present disclosure also relates to a computer-implemented method of determining a dynamic examination process for medical examinations. The method comprises determining a dynamic examination process based on a pathology hypothesis using a predefined algorithm. Furthermore, the method' comprises the dynamic examination process being associated with obtaining medical data, wherein the dynamic examination process is adapted during its execution, based on the obtained medical data.

The present disclosure also relates to a data processing system comprising means for carrying out the method according to any examples of the present disclosure.

The present disclosure also relates to a computer program comprising instructions which, when the program is executed by a computer, cause the computer to carry out the method according to any examples of the present disclosure.

In case the method operations may comprise any aspects (for example physical) which go beyond a mere data processing (for example an ultrasound signal processing), the computer program may further comprise computer-readable instructions which when executed by a data processing system cause any external elements of a system (for example an ultrasound transducer device) to carry out these operations.

The present disclosure also relates to a computer-readable medium comprising instructions which, when executed by a computer, cause the computer to carry out the method according to any examples of the present disclosure.

A dynamic examination process may have numerous advantages in medical imaging. The dynamic examination process may, for example, provide access to personalized care and improve the accuracy of ultrasound exams by leveraging optimal tools, best adapted and targeted tools (for instance tools that are identified based on the pathology hypothesis assumption) that are automatically called by the dynamic examination process. The dynamic examination process may also improve subject management by providing targeted exams and reducing healthcare costs. The dynamic examination process may increase productivity by optimizing workflow and making exams faster. In addition, the dynamic examination process may increase standardization and exam reproducibility by providing consistent modes and measurements. The dynamic examination process may improve documentation by automatically incorporating pathology-related reference values and publication sources (or targeted publication sources) into reports. The dynamic examination process may also reduce operator dependency and increase exam reproducibility, optionally filling the gap between expert and non-expert users (for example technicians or non-expert physicians) through automation and systematization. Finally, dynamic examination process may increase the reproducibility of specific process-defined sequences over time for follow-up exams (for example medical exams and/or ultrasound exams).

The processing unit may be a component of electronic devices that may be responsible for carrying out computational tasks. There may be different types of processing units, each designed for specific purposes. A processing unit may be or may comprise a Central Processing Unit (CPU), Graphics Processing Unit (GPU), Digital Signal Processor (DSP), Field-Programmable Gate Array (FPGA), and/or Application-Specific Integrated Circuit (ASIC). The CPU may be the primary processing unit in a computer that may be responsible for executing most of the instructions and calculations required by the computer. GPUs may be specialized processing units that may be designed to handle the complex calculations required for rendering graphics and video. DSPs may be specialized processing units that may handle signal processing tasks, while FPGAs may be reconfigurable processing units that may be programmed to perform various computational tasks. ASICs may be customized processing units designed to perform a specific set of tasks, optionally making them highly efficient and effective for their intended purpose. These processing units may be found in a wide range of electronic devices, such as medical electronic devices. Medical electronic devices may include computers, smartphones, and other digital devices, optionally enabling these devices to perform various computational tasks efficiently and accurately. The method according to the present disclosure may also run on a remote, cloud based and/or virtual server.

It is intended that combinations of the above-described elements and those within the specification may be made, except where otherwise contradictory.

It is to be understood that both the foregoing general description and the following detailed description are exemplary and explanatory only, are provided for illustration purposes and are not restrictive of the disclosure, as claimed:

The accompanying drawings, which are incorporated in and constitute a part of this specification, illustrate examples of the disclosure and together with the description, and serve to support and illustrate the principles thereof.

### BRIEF DESCRIPTION OF THE DRAWINGS

Fig. 1 schematically shows a dynamic examination process 100 for medical examinations of a subject according to examples of the present disclosure.
Fig. 2A shows another schematic example of a dynamic examination process 100 according to examples of the present disclosure.
Fig. 2B shows the dynamic examination process 100 of Fig. 2A wherein the architecture is adapted.
Fig. 3A shows a first example of a dynamic examination process 100 for an assessment of liver disease comprising several stages (S1 to S8).
In Fig. 3B, the dynamic examination process 100 selects entities associated with the first four stages of the assessment process of Fig. 3A (S1, S2, S3, and S4).
Fig. 3C shows the dynamic examination process 100, wherein entities associated with the further stages S5 and S6 of the assessment process of Fig. 3A are selected.
Fig. 3D shows the dynamic examination process 100, wherein an entity associated with the further stage S7 of the assessment process of Fig. 3A is selected.
Fig. 3E shows the dynamic examination process 100, wherein an entity associated with the further stage S8 of the assessment process of Fig. 3A is selected.
Fig. 3F shows a second example of a dynamic examination process 100' for an assessment of liver disease comprising several stages.
Fig. 4A shows a schematic flow chart of determining a dynamic examination process 100 using an AI model according to examples of the present disclosure.
Fig. 4B shows a schematic drawing of a clinical assessment process according to examples of the present disclosure.
Fig. 5A shows a schematic drawing of an ultrasound system 10 according to examples of the present disclosure.
Fig. 5B shows a schematic drawing of an exemplary dynamic examination process 100 for the ultrasound system 10 of Fig. 5A.

### DESCRIPTION OF THE DRAWINGS

Reference will now be made in detail to examples of the disclosure, examples of which are illustrated in the accompanying drawings. Wherever possible, the same reference numbers will be used throughout the drawings to refer to the same or like parts.

In general, the present disclosure, as explained in more detail in context of the following examples, refers to a system and method for determining a dynamic examination process. A system for determining a dynamic examination process may involve the use of technology to develop a systematic and/or highly customized approach for obtaining reliable, comprehensive and meaningful medical data of a subject. This system is, for example, further designed to streamline the process of obtaining medical data and of medical decision-making and treatment, while reducing the potential for errors and improving the efficiency of healthcare delivery.

With the aid of a dynamic examination process according to the present disclosure, it is possible to process large volumes of medical data, resulting in improved accuracy of diagnoses and better recommendations for treatment in the medical field. Furthermore, computational tools may enable automate routine tasks and decision-making processes, freeing up healthcare professionals to focus on more complex and critical tasks, improving efficiency, and reducing costs. Personalized care may be made possible with computational tools by analyzing patient data to provide tailored treatment recommendations based on a patient's medical history and current condition. The dynamic examination process may help reduce errors and improve patient safety by providing healthcare professionals with standardized and consistent treatment guidelines, leading to improved patient outcomes. Overall, the combination of the dynamic examination process with computational tools has the potential to revolutionize the field of medicine, providing standardized and efficient care, improving patient outcomes, and reducing costs while maintaining patient safety.

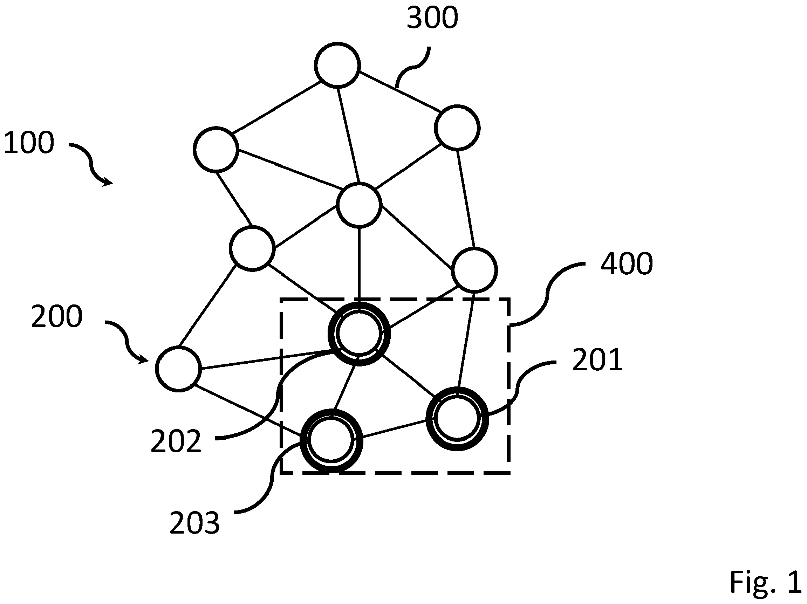

Fig. 1 schematically shows a dynamic examination process 100 for medical examinations of a subject according to examples of the present disclosure. The dynamic examination process 100 define entities 200, which are capable of executing a task. The entities 200 are interconnected and capable of communicating with each other through a digital communication channel 300 established according to the dynamic examination process 100. The dynamic examination process 100 is designed to define how the entities 200 interact with each other during the examination process, and the communication channel 300 facilitates the transfer of data between entities 200. The dynamic examination process 100 enables the execution of medical examinations of a subject. The entities 200 can be dynamically adapted based on the obtained medical data during the execution of the dynamic examination process 100, allowing for real-time adjustments and optimization of the examination process. The digital communication channel 300 facilitates communication between the entities 200, allowing for the exchange of information and the coordination of tasks.

The acquisition mode 400, which is responsible for acquiring medical data from the subject, is associated with a group of entities 201, 202, and 203. The entities 201, 202, and 203 are capable of executing specific tasks related to the acquisition of medical data, and are interconnected and communicate with each other through the digital communication channel 300 established by the dynamic examination process 100. The specific tasks may include guidance and measurement tools, as well as tools for assessing tissue characteristics such as stiffness and elasticity and visco-elasticity.

At the captured time, entities 201, 202, and 203 associated with the acquisition mode 400 are activated. The entities 201, 202, and 203 associated with the acquisition mode 400 depend on each other.

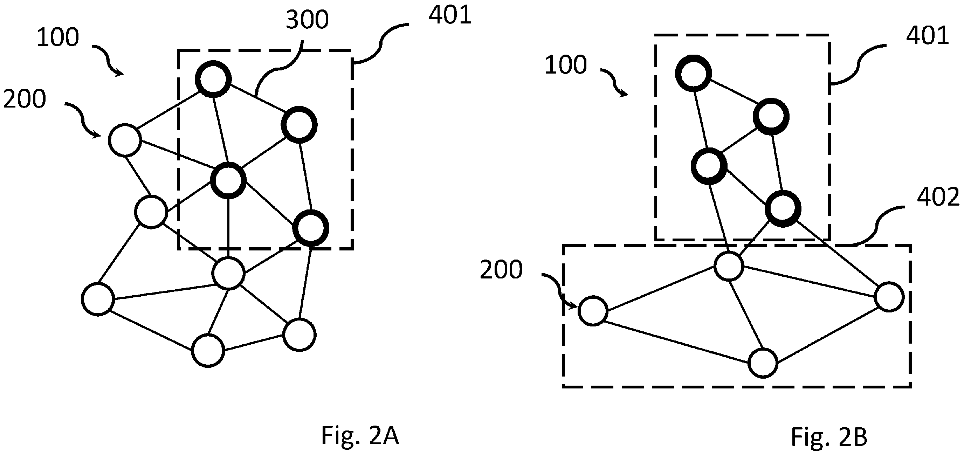

Fig. 2A shows another schematic example of a dynamic examination process 100 according to examples of the present disclosure. The dynamic examination process 100 comprises a group of entities 401. New information (i.e. medical data of a subject) is obtained by the group of entities 401 during the execution 100 of the dynamic examination process, so that the architecture has to be adapted by dynamically changing the entities and the communication channels 300 between different entities.

Fig. 2B shows the dynamic examination process 100 of Fig. 2A, wherein the architecture is adapted.

The dynamic examination process 100 comprises the group of entities 401, which is not changed in fig. 2B in view of Fig. 2A. A new group of entities 402 is added to the dynamic examination process 100 based on the obtained medical data. Entities 200 that do not belong to either group 401 or 402 have been removed, resulting in a new architecture for the dynamic examination process 100. The dynamic adaptation of the architecture may help to ensure that the dynamic examination process 100 remains efficient and effective in achieving its intended goals, even in the face of changing or uncertain medical data.

In other words, the dynamic examination process 100 is adapted based on medical data obtained by a first group of entities 401. A second group of entities 402 is adapted. Furthermore, the first and second group of entities 401, 402 may be associated with different acquisition modes.

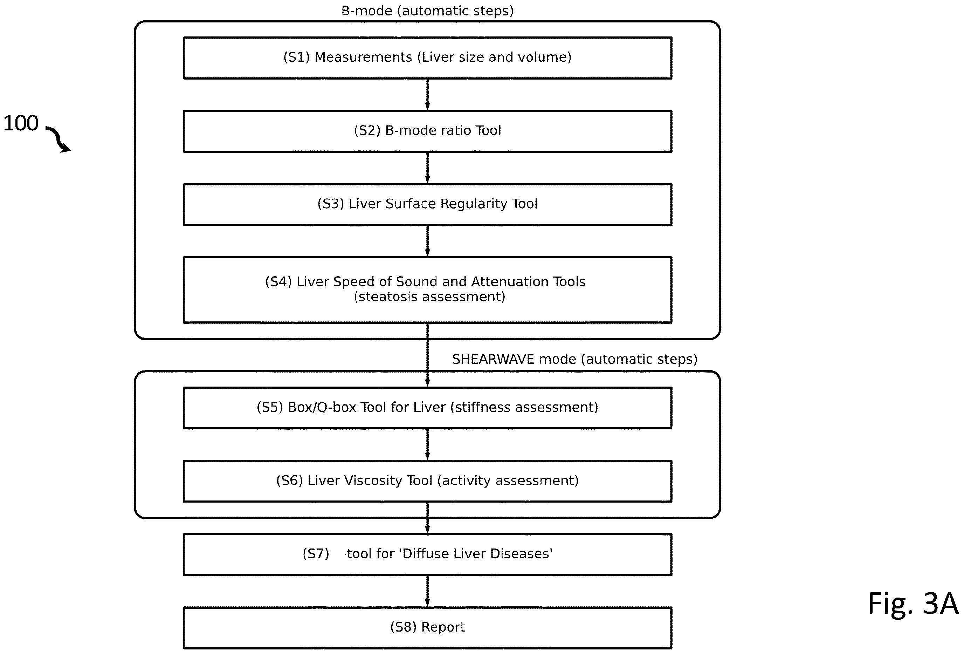

Fig. 3A shows a first example of a dynamic examination process 100 for an assessment of liver disease comprising several stages (S1 to S8).

The first stage S1 may involve using automatic measurements to assess the liver size and volume. In the second stage S2, a B-mode ratio tool may be used. For example, the B-mode ratio tool may determine a relative brightness between two different mediums or two different areas in a medium, for instance between Liver and Kidney. The ratio may be computed on B-mode images between the echogenicity of for example the hepatic parenchyma and the renal cortex. The third stage S3 comprises the application of a liver surface regularity tool. Said tool may be an example of an intelligent analyzing and/or processing component, as described above. In a fourth stage S4, liver speed of sound and attenuation tools for steatosis assessment are used. The four stages S1, S2, S3, and S4 may be four independent stages relying on (or being based on) B-mode data.

In the fifth stage S5, a tool for liver stiffness assessment is applied (for example box/Q-box ^{®} tool). Said tool may be a further example of an intelligent analyzing and/or processing component, as described above. A sixth stage S6 comprises a liver viscosity tool for activity assessment. Stages S5 and S6 are for example two stages relying on ShearWave^{™} Elastography (SWE) data. A seventh stage S7 may comprise the use of a component for "Diffuse Liver Diseases" which may enable multi-parametric analysis, optionally taking into account a stiffness value, a visco-elasticity value, and confounding factors analysis. The analysis in this stage may include anatomy, fibrosis, steatosis, activity staging, risk, and recommendations on subject management. Said component of stage S7 may receive as input the output of the components of stages S3 and S5.

Finally, the assessment may conclude with an eighth stage S8 comprising a report that may be generated based on the output of the component for "Diffuse Liver Diseases" of stage S7 and optionally includes specific references to pathology (for example any data allowing assessing the validity of the initial pathology hypothesis).

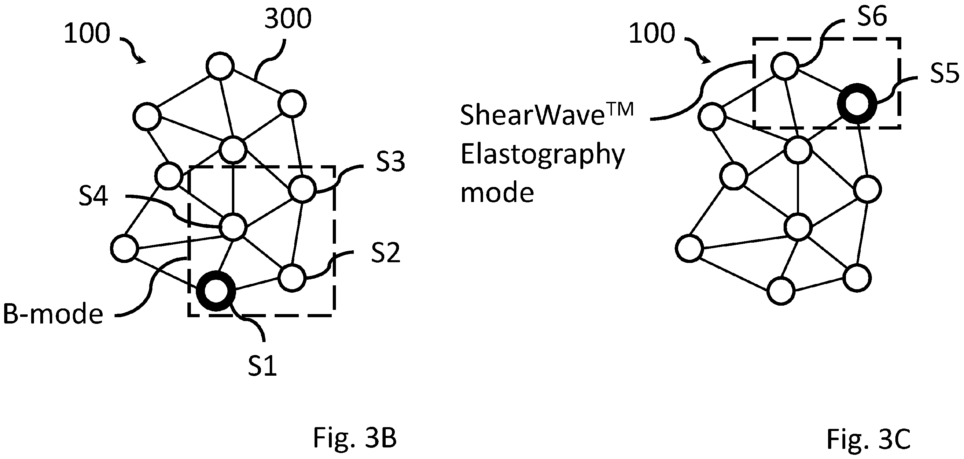

As shown in the following fig. 3B to 3E, it may be possible that the dynamic examination process is adapted during its execution by selecting at each instance of progress a suitable entity, and optionally providing information, such as already obtained medical data to this entity. This entity may then be activated, for example for executing or controlling a task. Accordingly, in this example, the architecture of the dynamic examination process is not adapted (for example by adding or removing entities or communication channels) but by a respective selection of predefined entities. Still, the selected entities may be configured based on the already obtained medical data and/or the pathology hypothesis.

In Fig. 3B, the dynamic examination process 100 selects entities associated with the first four stages of the assessment process of Fig. 3A (S1, S2, S3, and S4) through the use of entities 200.

Fig. 3C shows the dynamic examination process 100, wherein entities associated with the further stages S5 and S6 of the assessment process of Fig. 3A are selected.

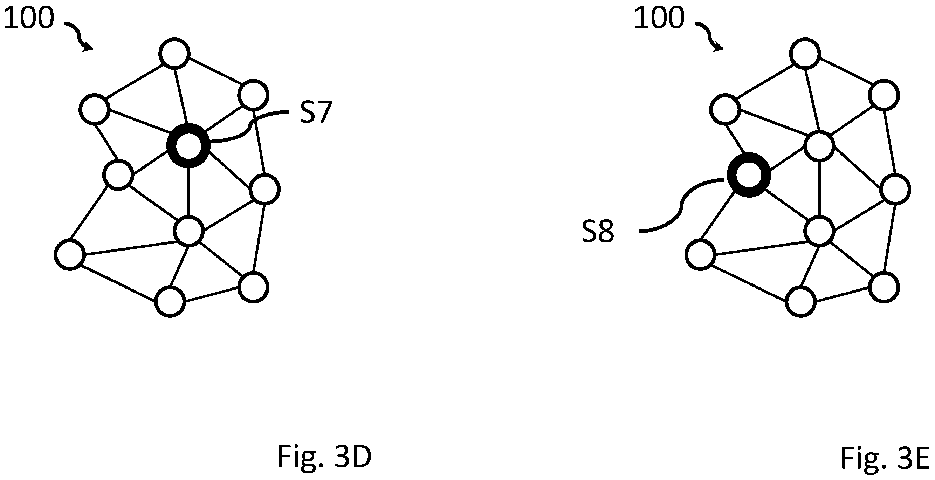

Fig. 3D shows the dynamic examination process 100, wherein an entity associated with the further stage S7 of the assessment process of Fig. 3A is selected. In stage S7 the component for "Diffuse Liver Diseases" is applied.

Fig. 3E shows the dynamic examination process 100, wherein an entity associated with the further stage S8 of the assessment process of Fig. 3A is selected. The stage S8 comprises a report that may be generated based on the output of the component for "Diffuse Liver Diseases" of stage S7 and optionally includes specific references to pathology (for example any data which allow assessing the validity of the initial pathology hypothesis).

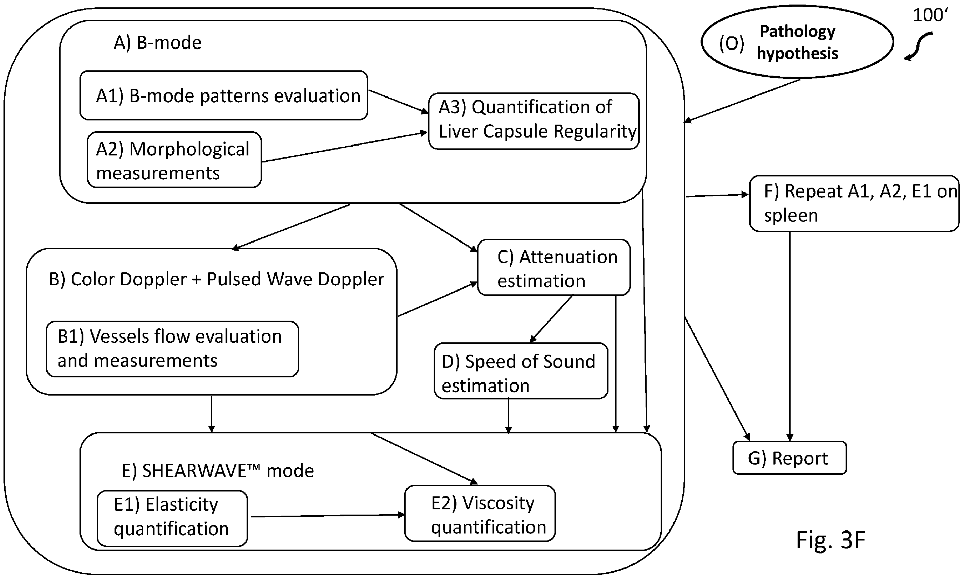

Fig. 3F shows a second example of a dynamic examination process 100' for an assessment of liver disease comprising several stages.

Conventionally, an ultrasound imaging system may be provided with a "factory" protocol, for instance for abdominal examination. Since the steps of such protocols are predefined, these steps or the protocol in general does not adapt to a particular subject which is examined. Hence, on the one hand, there is the risk that the predefined protocol includes examination steps, which are unnecessary in view of a particular subject. Accordingly, the predefined protocol can imply a waste of time for both obtaining the medical data and for analyzing the examination results. On the other hand, there is the risk that the predefined protocol lacks any steps which would be necessary to obtain relevant information about a particular subject. Accordingly, a final examination result may be less accurate or even incorrect.

In view of these drawbacks the present disclosure provides a dynamic examination process which may auto-adapt to a particular subject while obtaining the medical data. For example, as described in context of fig. 3F, an ongoing ultrasound examination process can be auto-adapted in real-time during its execution, as a function of obtained information of the subject. The stages described in the following are not necessarily sequential. Instead, the stages may also be performed simultaneously. Moreover, in case the stages are performed sequentially, their order may be different than in the example of fig. 3F. In addition, some of the shown stages may be skipped, in case they are determined to be not necessary in view the results of already performed stages. At the same time, other stages might also be added which are not shown in the present example, in case they are determined to be required in view the results of already performed stages.

In stage O, a pathology hypothesis may be provided, for example as manual input or based on a machine-based determination, as described above. The pathology hypothesis may also be associated with a pathology family. For example, it may be assumed that the pathology family is a diffuse liver disease.

In stage A1, B-mode patterns may be evaluated based on obtained B-mode data of the liver. Such patterns may include for example homogeneity and/or brightness patterns information associated with the liver.

In stage A2, Morphological measurements may be performed based on obtained B-mode data of the liver. For example, the liver size or a region of interest in the liver may be measured.

In a stage A3, the liver capsule regularity may be quantified based on obtained B-mode data of the liver. This stage may depend on the results of stages A1 and A2. In other words, stage A3 may only be performed, in case the stage is determined to be necessary in view of the results of stages A1 and A2. For example, in case in stage A1 a decreased homogeneity of the liver is determined, and/or in case in stage A2 an increased liver size is measured, it may be determined that stage A3 needs to be performed.

The stages stage A1 to A3 may be part of a B-mode stage A.

These stages A1 to A3, as well as the stages B to G described in the following may be performed at least partially automatically by an ultrasound system, and/or the system may guide a user when performing the stages, for instance to place an ultrasound probe at a particular position of the subject.

In stage B1 a vessel flow may be evaluated and measured based on color doppler data and/or pulsed wave doppler data. This stage may depend on the results of stages A1, A2 and A3. In one example, the evaluation and measurement process of stage B1 may be finetuned based on these results of stages A1, A2 and A3, for example by adjusting a region of interest in the liver, selection of a particular ultrasound probe, selection of settings, or selection of measurement types (for instance mean velocity vs resistive index). For instance, the region of interest for obtaining color doppler data and/or pulsed wave doppler data may be adjusted based on measurements made in stage A2.

In case the results of stages A and B indicate that there is no diffuse liver disease, the dynamic examination process 100' may be stopped. In other words, the further stages C to F may be omitted and optionally a report may be generated, see stage G.

In stage C an attenuation coefficient may be estimated based on the results of stages A and B. For example, depending on the results of stages A and B. (for instance B-mode data and color doppler data and/or pulsed wave doppler data), it may be determined whether additionally the measurement of an attenuation coefficient is required to improve the accuracy of the ultrasound exam and/or of the examination results. According to a further example, the attenuation coefficient may be measure for one or several regions of interest in the liver which have been identified in the stages A and B.

In stage D a speed of sound coefficient may, be estimated based on the result of stage C. For example, depending on the results of stage, it may be determined whether additionally the measurement of an speed of sound coefficient is required to improve accuracy of the ultrasound exam and/or of the examination results. According to a further example, the estimated attenuation coefficient may be taken into account in a speed of sound estimation process. Moreover, the speed of sound coefficient may be estimated for the same regions of interest as the attenuation coefficient..

In stage E2 a viscosity quantification of the liver may be performed based on obtained ShearWave^{™} elasticity dispersion data of the liver. This stage may depend on the results of stages A to D and additionally of stage E1. For example a viscosity model may be used which takes into account the quantified elasticity of the liver.

The stages stage E1 and E2 may be part of a ShearWave^{™} elasticity stage

In stage F the stages A1, A2 and E1 may be applied to the spleen of the subject. This stage may depend on the results of stages A to E. For example, spleen assessment can allow detection of portal hypertension that is related to high stages of fibrosis. Assessing the spleen may allow at least one of: evaluating portal hypertension, defining the need of further exams of other organs than liver and spleen, and correcting liver elasticity depending on the presence or not and the severity of the spleen stiffness. By applying the stages A1, A2 and E1 to the spleen, the same tools and features may be used for spleen assessment as on the liver assessment.

In stage G a report may be generated based on the results of the stages A to F. The report may also comprise an assessment of the initially assumed pathology hypothesis. For this purpose, a multi-parametric analysis process may be performed, optionally corrected by confounding factors associated with the data obtained in the different stages. The report may for example comprise indicators for staging of steatosis, inflammation, congestion of the liver, etc. Furthermore, the report may comprise an indicator for a stratification level of the liver. For example, a classification in a category may be automatically determined, for instance a classification regarding a risk of decompensation of the liver. Furthermore, the report may comprise an indicator associated with a health risk, for example of esophageal varices, and optionally with a recommendation on patient management, for instance of performing an echo-endoscopy.

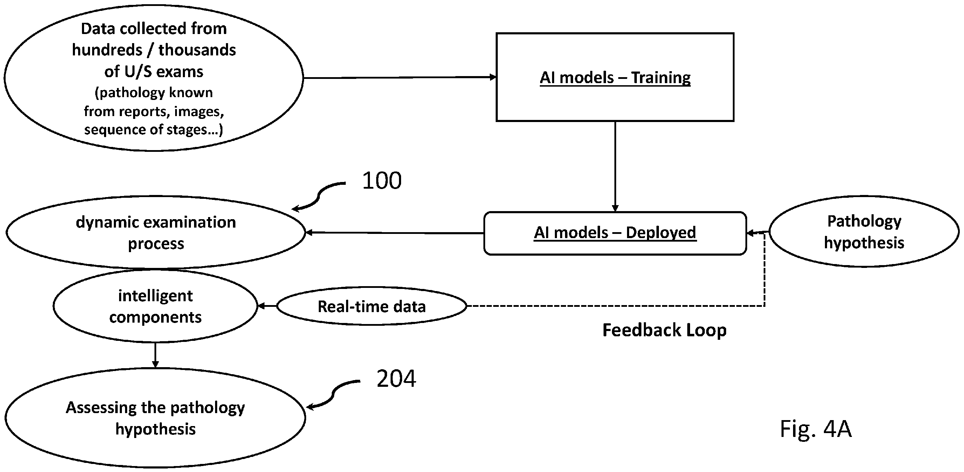

Fig. 4A shows a schematic flow chart of determining a dynamic examination process 100 using an AI model according to examples of the present disclosure. The predefined algorithm may be developed as an artificial intelligence (AI) model that has been trained on data from hundreds or thousands of ultrasound (U/S) exams, including information about known pathologies from reports, images, sequence of stages, and other relevant factors. This training allows the model to learn patterns and correlations in the data, and use that knowledge to make accurate predictions and recommendations for future exams. The AI model may use the pathology hypothesis to determine a customized dynamic examination process 100 for the ultrasound examination. The dynamic examination process 100 may comprise one or several components. The dynamic examination process 100 defines one entity 204 configured to assess the pathology hypothesis or a particular member of a pathology family as a function of the obtained medical data.

Real-time data acquired by an entity of the dynamic examination process 100 (i.e. obtained medical data of the subject) may be used in a feedback loop. For that, the real-time data may be used as input for the AI model. Accordingly, the AI model may adapt the dynamic examination process based on the real-time data.

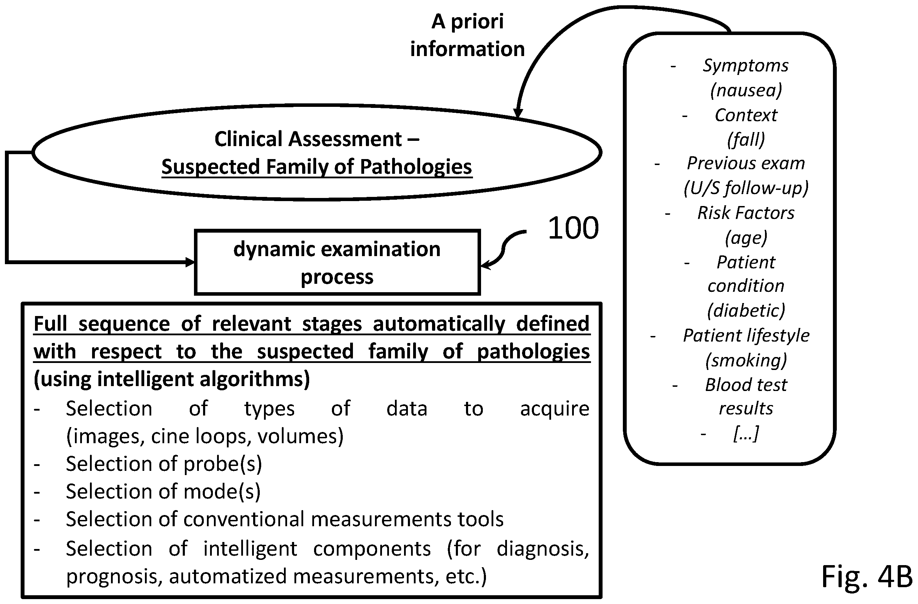

Fig. 4B shows a schematic drawing of a clinical assessment process according to examples of the present disclosure. A clinical assessment process, which may involve evaluating a subject's medical history, symptoms, and physical examination, can be used to identify a suspected family of pathologies. A clinical assessment process may be based on various types of a priori information, such as symptoms the subject is experiencing, the context in which the examination is being performed, the subject's medical history, risk factors, the subject's overall health condition, lifestyle factors, and the results of blood tests or other diagnostic tests. This information can then be used to determine a dynamic examination process 100 for a medical examination using an ultrasound system and an AI model trained on data collected from hundreds or thousands of previous ultrasound exams.

The dynamic examination process 100 may include a sequence of relevant stages that are automatically defined based on the suspected family of pathologies, and this can be achieved using intelligent predefined algorithms. The dynamic examination process 100 can also involve selecting the types of data to acquire, such as images, cine loops, or selected 3D (i.e. three-dimensional) volumes of a scanned medium of the subject (for example, in case of 3D imaging), as well as the appropriate probe(s) to use. A volume may be a sum of 2D (i.e. two-dimensional) slices as used in conventional 2D ultrasound imaging methods. Other aspects that may be determined by the dynamic examination process include the mode(s) to be used, the measurement tools to be employed, and the intelligent components that will be leveraged for different purposes, such as diagnosis, prognosis, automatized measurements, etc.. By incorporating all of these factors into the dynamic examination process 100, it is possible to standardize the ultrasound exam and improve its accuracy, efficiency, and productivity.

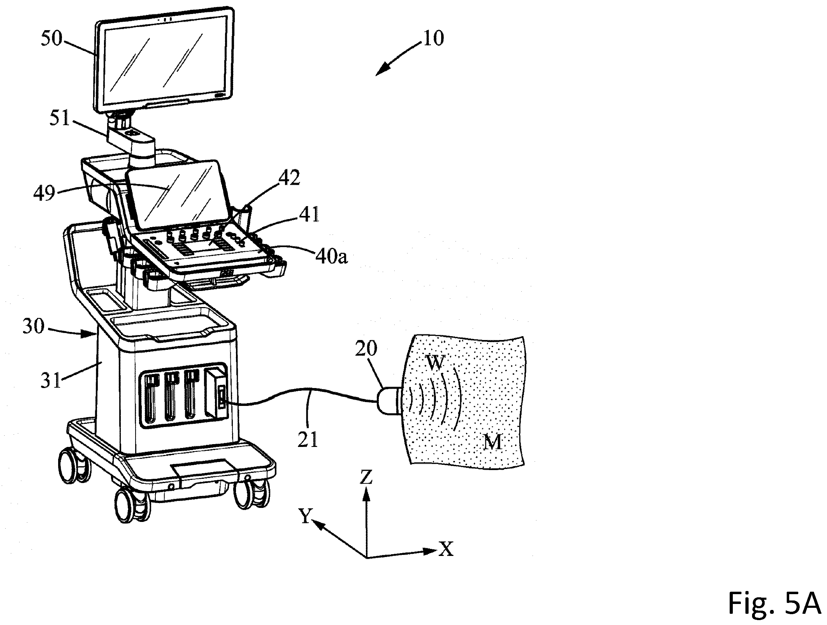

Fig. 5A shows a schematic drawing of an ultrasound system 10 according to examples of the present disclosure. The ultrasound system 10 may be an example of a system for determining a dynamic examination process 100 for examinations.

The ultrasound imaging system 10 may comprise:
- a probe 20,
- a processing unit 30 for processing an image on the bases of signals received by the probe,
- a control panel 40a connected to the processing unit, said control panel at least comprising buttons 41 and a touch pad 42, and
- a display 50 for visualizing the image.

The probe 20 may be associated , for example connected to the processing unit 30 via a cable 21 or via a wireless connection, and it is able to emit ultrasound waves W into a medium M and/or to receive ultrasound waves W from the medium M, said received ultrasound waves being consequent or resulting from reflections of the emitted ultrasound waves on diffusing particles present in medium M. Probe used for emitting and receiving waves may be same or separated.

The display screen 50 may be a screen for visualizing the image processed by the processing unit 30. The display 50 may also visualize other information such as scales used in the image, values map, configuration information for the processing or any information such as help information or contextual gesture help for the touch pad 42.

The display screen may by articulated on a support arm 51 for better positioning for the user. The display screen is usually a high definition screen of a great size (at least 20 inches) for better image visualization to the user.

The control panel 40a is for example a portion of the system casing 31, said portion comprising a panel casing having a substantially flat surface inclined towards the user for manipulation by one hand of said user. The control panel 40a may include a control panel display screen 49 for visualizing several configuration information or any information dedicated to the user.

The processing unit 30 is configured to determine a dynamic examination process 100 based on a pathology hypothesis using a predefined algorithm. That means the operator or user of the system 10, which may be for example a physician or a merely technically skilled person, first derives a pathology hypothesis. For example, in case the operator or user is a physician, said pathology hypothesis may be derived or determined based on observations of the subject to be examined and based on empirical knowledge. It is also possible that pathology hypothesis may be derived or determined automatically or at least semi-automatically using a computational tool (in particular in the exemplary case that the operator or user is a merely technically skilled person). The computational tool may be or may comprise software-based solution or (AI) algorithm. The computational tool may automatically process and/or analyze information available in the subject record. The computational tool may at least partially be external to the system 10, i.e. run at least partially on an external computing device. The operator then provides the pathology hypothesis as an input to the ultrasound imaging system 10 using the control panel 40a and/or the display screen 50. The processing unit 30 can then use the pathology hypothesis provided by the operator to automatically determine a dynamic examination process 100 using a predefined algorithm. The dynamic examination process 100 can be a full sequence of relevant steps or stages for the suspected family of pathologies. The operator can also monitor and adjust the dynamic examination process 100 as needed during the examination using the control panel 40a and the display screen 50.

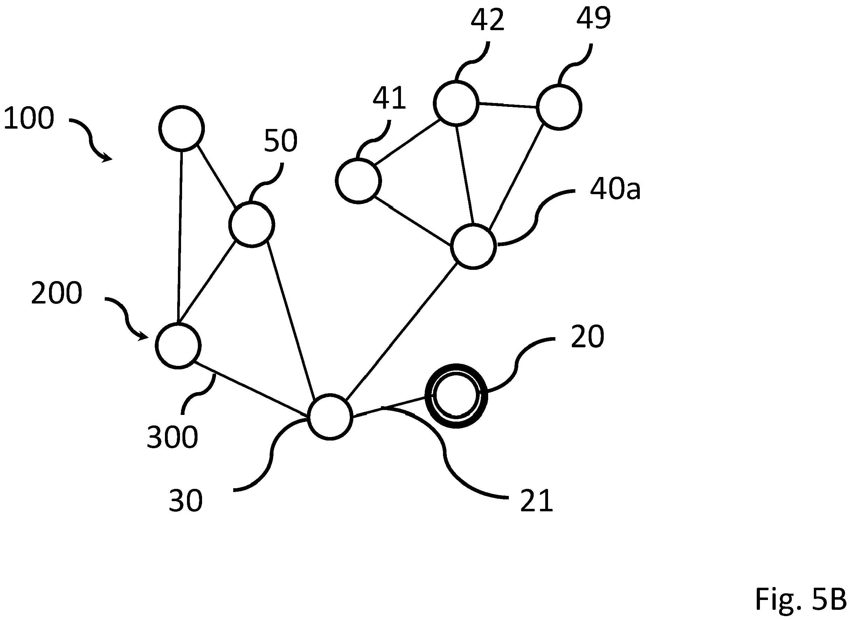

Fig. 5B shows a schematic drawing of an exemplary dynamic examination process 100 for the ultrasound system 10 of Fig. 5A. The dynamic examination process 100 may include a set of entities 200 that perform specific tasks, including the probe 20 that generates ultrasound signals and receives resulting echoes, the processing unit 30 that may convert the signals into an image, the control panel 40a that allows the operator to manually re-adjust system settings and image parameters (which may have been automatically set or adjusted by the system according to the present disclosure), and the display 50 that presents the resulting image. The entity associated to the control panel 40a is connected with the entities associated with the buttons 41, the touch pad 42, and the control panel display screen 49.

The dynamic examination process 100 may specify how these entities should be interconnected and how they should communicate with each other through a digital communication channel 300. The operator can follow the dynamic examination process 100, using the buttons 41 and touch pad 42 on the control panel to adjust the system settings and select the appropriate examination mode(s), resulting in an accurate and efficient medical examination.

The entities are adaptable during execution of the dynamic examination process 100 based on the obtained medical data. For example, if the physician uses a first type of probe 20, the processing unit 30 of the dynamic examination process 100 may analyze the obtained medical data and determine whether the probe 20 is appropriate for this examination. In response, the processing unit 30 of the dynamic examination process 100 may provide information to adapt the probe 20 by using a second type of probe 20. The probe 20 may be adapted accordingly. Furthermore, a machine parameter, such as gain, depth, and frequency, may be automatically adapted by the processing unit 30.

Another embodiment of a dynamic examination process may be an assessment of ageing-related musculoskeletal (MSK) disorders. The dynamic examination process may include B-mode and SWE-mode automatic stages. In the B-mode, an intelligent guidance component for MSK may be used to recognize typical patterns and acquire proper B-mode planes. The Echo Intensity Tool may be used for MSK to compute and position the region of interest (ROI) for myosteatosis assessment. In the ShearWave^{™} Elastography (SWE) mode, a guidance component may be used for determining MSK fibers alignment. The guidance component may further be used to acquire for example optimal SWE data by taking also into account determined MSK fibers alignment. the intelligent Box/Q-box component may be used for ShearWave^{™} speed assessment.

The dynamic examination process may include a component for "Ageing-Related MSK Disorders" which may perform a multi-parametric analysis that may include myosteatosis as a Sarcopenia biomarker. The component may diagnose and provide a prognosis for Sarcopenia. The report generated by the dynamic examination process may include specific references to the pathology and recommendations for subject management. The dynamic examination process may allow for the automated assessment of age-related MSK disorders and provides an accurate diagnosis and prognosis.

Another embodiment of a dynamic examination process may be the Evaluation of NACT (neoadjuvant chemotherapy) responsiveness for breast cancer comprising several stages. The first stage may involve Query/Retrieve for obtaining subject records and/or ultrasound data from previous exams. The second stage may be using B-mode with automatic annotations and body markers, and measurements of the lesion size. The third stage may involve SWE-mode with the Box/Q-box^{™} component for automated breast stiffness assessment, and a so-called TriVu^{™} mode which may provide the combination of Ultrasensitive Doppler with B-mode and SWE-mode. The fourth stage may involve the use of 3D PROBE in both B-mode and SWE-mode. The fifth stage may introduce a component specifically for "NACT responsiveness evaluation" which may include a multi-parametric analysis to evaluate the responsiveness of the chemotherapy. The last stage may be generating a report based on the analysis.

Throughout the description, including the claims, the term "comprising a" should be understood as being synonymous with "comprising at least one" unless otherwise stated. In addition, any range set forth in the description, including the claims should be understood as including its end value(s) unless otherwise stated. Specific values for described elements should be understood to be within accepted manufacturing or industry tolerances known to one of skill in the art, and any use of the terms "substantially" and/or "approximately" and/or "generally" should be understood to mean falling within such accepted tolerances.

The terms "record" and "receive" may be used synonymously throughout this disclosure unless denoted differently.

Although the present disclosure herein has been described with reference to particular examples, it is to be understood that these examples are merely illustrative of the principles and applications of the present disclosure.

It is intended that the specification and examples be considered as exemplary only, with a true scope of the disclosure being indicated by the following claims.

A reference herein to a patent document or any other matter identified as prior art, is not to be taken as an admission that the document or other matter was known or that the information it contains was part of the common general knowledge as at the priority date of any of the claims.

## Claims

1. A system (10) for determining a dynamic examination process, comprising a processing unit (30) configured to:
determine a dynamic examination process (100) based on a pathology hypothesis using a predefined algorithm,
the dynamic examination process (100) being associated with obtaining medical data,
wherein the dynamic examination process (100) is adaptable during its execution based on the obtained medical data.

2. The system according to any one of the preceding claims, wherein
the dynamic examination process (100) defines at least one entity (200, 201, 202, 203, 204, S1, S2, S3, S4, S5, S6, S7, S8) capable of executing or controlling a task.

3. The system according to claim 2, wherein
the at least one entity (200, 201, 202, 203, 204, S1, S2, S3, S4, S5, S6, S7, S8) is adaptable during execution of the dynamic examination process (100) based on the obtained medical data.

4. The system according to any one of the claims 2 and 3, wherein
the task and/or the entity (200, 201, 202, 203, 204, S1, S2, S3, S4, S5, S6, S7, S8) is associated with at least one of:
biomarkers,
computational tools,
analyzing and/or processing components,
measurement tasks, and/or measurement values,
acquisition modes,
probe types,
machine parameters, scan parameters,
acquisition process for obtaining medical data,
processing tools,
reporting tools, and
selecting medical data.

5. The system according to any one of the claims 2 to 4, wherein
the dynamic examination process (100) defines at least one predefined acquisition mode (400) of the medical device, the acquisition mode (400) being associated with a group of entities (401, 402).

6. The system according to any one of the claims 2 to 5, wherein
adapting the dynamic examination process (100) comprises:
based on the medical data obtained by a first entity (200, 201, 202, 203, 204, S1, S2, S3, S4, S5, S6, S7, S8) or a first group of entities (401, 402), reexecuting the first entity (200, 201, 202, 203, 204, S1, S2, S3, S4, S5, S6, S7,
S8) or first group of entities (401, 402) using at least one adapted machine parameter, and/or
based on medical data obtained by a first entity (200, 201, 202, 203, 204, S1, S2, S3, S4, S5, S6, S7, S8) or first group of entities (401, 402), selecting and/or adapting a second entity (200, 201, 202, 203, 204, S1, S2, S3, S4, S5, S6, S7, S8) or a second group of entities (401, 402),.

7. The system according to any one of claims 2 to 6, wherein
adapting the second entity comprises at least one of:
removing the second entity from the dynamic examination process (100),
adding the second entity to the dynamic examination process (100), and
adapting at least one parameter of the second entity.

8. The system according to any one of claims 2 to 8, wherein
at least one entity (200, 201, 202, 203, 204, S1, S2, S3, S4, S5, S6, S7, S8) is executable using a medical device.

9. The system according to any one of claims 2 to 9, wherein
the dynamic examination process (100) defines a group of interconnected entities (401, 402), wherein at least two entities (200, 201, 202, 203, 204, S1, S2, S3, S4, S5, S6, S7, S8) of the group depend on each other.

10. The system according to the preceding claim, wherein
at least two entities of the group of interconnected entities (200, 201, 202, 203, 204, S1, S2, S3, S4, S5, S6, S7, S8) are capable of communicating with each other through a digital communication channel (300), optionally established according to the dynamic examination process (100).

11. The system according to any one of the preceding claims, wherein
the obtained medical data comprise at least one of:
data acquired from the subject in real-time,
medical examination data, and
data acquired by at least one medical device, optionally including an ultrasound system (10).

12. The system according to any one of the preceding claims, wherein
the dynamic examination process (100) is adaptable in real-time.

13. The system according to any one of the preceding claims, wherein
the pathology hypothesis comprises a hypothesis of a pathology family, and/or the dynamic examination process (100) defines at least one entity (200, 201, 202, 203, 204, S1, S2, S3, S4, S5, S6, S7, S8) configured to:
assess the pathology hypothesis or a particular member of a pathology family as a function of the obtained medical data.

14. The system according to any one of the preceding claims, wherein
the predefined algorithm is or comprises an artificial intelligence (AI) model, and/or
at least one entity is or comprises an artificial intelligence (AI) model.

15. A computer-implemented method of determining a dynamic examination
process (100) for medical examinations of a subject, the method comprising:
determining a dynamic examination process (100) based on a pathology hypothesis using a predefined algorithm,
the dynamic examination process (100) being associated with obtaining medical data,
wherein the dynamic examination process (100) is adapted during its execution, based on the obtained medical data.

16. A computer program comprising instructions which, when the program is
executed by a computer, cause the computer to carry out the method according to any one of the preceding method claims.
